# FASCICULE DE BREVET EUROPEEN

(11) **EP 0 346 241 B1**
(45) Date de publication et mention de la délivrance du brevet: **01.03.1995**
(21) Numéro de dépôt: 89401605.4
(22) Date de dépôt: 09.06.1989
(51) Int. Cl.: C12N 9/64, A61K 38/48

(54) **Procédé de préparation d'une fraction concentrée en facteur Vlla et son application à titre de médicament**
Verfahren zur Herstellung einer an Faktor-VIIa angereicherten Fraktion und ihre Verwendung als Arzneimittel
Process for preparing a factor VIIa fraction, and its use as a medicament

(30) Priorité: 09.06.1988 FR 8807687
(43) Date de publication de la demande: 13.12.1989
(73) Titulaire: FONDATION NATIONALE DE TRANSFUSION SANGUINE, 75739 Paris Cédex 15 (FR)
(72) Inventeur: Chabbat, Jacques, F-75017 Paris (FR); Pejaudier, Lucette, F-75016 Paris (FR); Steinbuch, Marion, F-78180 Voisins Le Bretonneux (FR)
(74) Mandataire: Warcoin, Jacques

(56) Documents cités:
- WO-A-83/00016
- US-A- 4 470 969
- US-A- 4 473 553
- US-A- 4 637 932
- CHEMICAL ABSTRACTS, vol. 91, no. 23, 3 décembre 1979, page 399, no. 190421n, Columbus, Ohio, US; U. SELIGSHON et al.: "Activation of human factor VII in plasma and in purified systems. Roles of activated factor IX, kallikrein, and activated factor XII", & J. CLIN. INVEST. 1979, 64(4), 1056-65

## Description

La présente invention concerne un procéde de préparation d'une fraction riche en facteur VIIa et sensiblement dépourvue de facteur VIII, facteur IX, facteur X et facteur II, ainsi que son application à titre de médicament pour le traitement des hémophiles A ayant un inhibiteur circulant.

Le facteur VII est impliqué dans la voie extrinsèque de la coagulation sanguine. Cette protéine vitamine K-dépendante est un proenzyme du poids moléculaire 50 000 daltons qui peut être activé par différents activateurs et en particulier le facteur XIIa (facteur Hageman activé).

L'activité biologique du facteur VII est dépendante de l'interaction avec une protéine non présente dans la circulation sanguine : le facteur tissulaire (F.T.) appelé également thromboplastine tissulaire. Ce facteur est constitué d'une partie protéique de poids moléculaire 45 000 daltons et d'une partie phospholipidique. Le complexe formé FVII-FT et/ou FVIIa-FT en présence d'ions calciums transforme le facteur X (proenzyme) en facteur X activé (enzyme). Ce dernier transforme la prothrombine en thrombine, enzyme responsable de la formation du caillot en générant la fibrine à partir du fibrinogène.

Les deux formes FVII et FVIIa sont capables de former un complexe calcium dépendant avec le facteur tissulaire avec la même affinité. Cependant, le complexe FVIIa-FT a une activité coagulante 25 à 100 fois plus élevée que le complexe FVII-FT.

Dans le traitement des hémophiles A ayant un inhibiteur circulant (présence d'anticorps dirigés contre le facteur VIII), des fractions dites "activées" ont été et restent actuellement utilisées comme thérapie substitutive pour rétablir une hémostase normale chez ces malades.

Ces fractions sont constituées des facteurs du complexe prothrombique dans leurs formes native et activée ; on observe en effet la présence de IXa, Xa, XIa, XIIa et VIIa et de traces de thrombine. L'agent ou les agents responsables de l'effet court-circuitant l'inhibiteur anti FVIII (effet by-pass) ne sont pas, à ce jour, caractérises.

Le facteur VII activé est un bon candidat, en effet il est présent dans ces préparations : AUTOPLEX (Hyland Travenol) et FEIBA (Immuno AG). D'autre part, en 1983, U. Hedner et W. Kisiel ont pu démontrer cliniquement l'efficacité du facteur VIIa hautement purifié sur deux hémophiles A ayant un titre élevé d'inhibiteurs du FVIII (U. Hedner and W. Kisiel, 1983, J. Clin. Invest., 71, p1836-1841).

Comparé aux autres facteurs activés, le FVIIa présente l'intérêt de pouvoir agir localement selon la disponibilité de son cofacteur, le facteur tissulaire, qui est libéré après lésion tissulaire, comme par exemple lors de traumatisme (hémarthroses graves, interventions chirurgicales, extractions dentaires, etc.). Un autre intérêt à l'utilisation du facteur VIIa comme by-pass est sa demi-vie très courte, soit environ 4 heures, alors que les autres facteurs du complexe prothrombique ont une demi-vie plus longue aboutissant à l'accumulation de certains facteurs (II et X notamment) lors des traitements d'entretien suivant les interventions chirurgicales.

La présente invention concerne un procédé d'obtention d'un concentré de FVII activé, inactivé viralement sans préjudices notables sur son activité coagulante.

Le concentré ainsi obtenu présente l'avantage, comparé à d'autres fractions activées, de ne contenir que très peu de FVIII antigène, d'une part, et des quantités faibles de facteurs IX, X et II, d'autre part.

Ce concentré de FVIIa est obtenu après adsorption puis élution d'un sous-produit du fractionnement de protéines plasmatiques contenant le facteur VII et VIIa et d'autres protéines telles les facteurs IX, X et II, notamment d'une sous-fraction non utilisée dans la préparation du complexe prothrombique (PPSB : P = prothrombine ou FII, P = proconvertine ou FVII, S = facteur STUART ou FX et B = facteur antihémophilique B ou FIX). Cette sous-fraction est appelée prééluat du PPSB. Il s'agit, de préférence, d'un sous-produit de fractionnement n'utilisant pas de solvant organique, riche en FVII et appauvri en facteurs II, IX et X.

Plus particulièrement, la présente invention concerne un procédé de préparation d'une fraction riche en facteur VIIa, caractérisé en ce que :
- on fait adsorber une fraction contenant du facteur VII et VIIa, notamment le prééluat du PPSB, par un absorbant ayant une affinité sélective pour les protéines se fixant au calcium choisi parmi les sels de métal divalent insoluble dans l'eau,
- on élue les protéines fixées par addition d'un tampon contenant des sels solubles capables de déplacer les protéines fixées,
- on récupère l'éluat concentré en facteur VIIa.

Bien qu'il soit possible d'utiliser le procédé selon l'invention pour obtenir des fractions enrichies en FVIIa à partir de fractions très diverses contenant le FVIIa, il est particulièrement intéressant d'utiliser la fraction surnageant de la cryoprécipitation lors de la préparation du PPSB.

Plus précisément, le prééluat est tout d'abord concentré, par exemple par ultrafiltration en présence d'un inhibiteur de la kallicréine, l'aprotinine, ajouté à raison de 30 U/ml. On amène la solution de prééluat à une concentration de 20 à 80 g/l et à une osmolarité de 0,12 à 0,2 M NaCl tamponnée à pH de 7 à 9. L'adsorbant que l'on utilise de préférence est le phosphate tricalcique connu pour retenir les facteurs vitamine K-dépendant et en particulier le facteur VII, notamment sous forme d'hydroxyapatite ou de brushite, bien qu'il soit possible d'utiliser d'autres sels tels que les citrates. Le phosphate tricalcique est ajouté entre 5 et 15 g/l de solution protéique. La suspension est mise en agitation à température ambiante, soit 15 à 30°C, pendant 20 minutes à 1 heure puis centrifugée. Les étapes d'adsorption et d'élution peuvent également être mises en oeuvre sur colonne.

Le surnageant de centrifugation (ou non adsorbé) est écarté et le support est lavé avec une solution contenant 0,1 à 0,2 M NaCl tamponnée à pH de 7 à 9, le volume utilisé pour le lavage étant le même que celui mis en oeuvre lors de l'adsorption.

Cette deuxième suspension est agitée pendant 15 minutes à 1 heure à une température de 15 à 30°C puis centrifugée dans les mêmes conditions que précédemment, par exemple une centrifugeuse à alimentation continue (type Cepa ou Sharples), le débit est de 40 ± 5 l/h).

Ainsi, le deuxième surnageant est éliminé et le culot remis en suspension dans la solution éluante ayant pour volume le 1/7ème de celui de départ. La solution éluante peut être soit un mélange de phosphates mono ou disodique de 0,2 à 0,3 M contenant du NaCl de 0,15 à 0,5 M, soit une solution de citrate trisodique de 0,1 à 0,25 M en présence de NaCl de 0,15 M à 0,5 M, dans les deux cas les solutions éluantes sont amenées à pH de 7 à 9.

Cette nouvelle suspension est agitée pendant 20 minutes à 1 heure à 15-30°C puis centrifugée. Cette fois, le culot est éliminé et le surnageant est récupéré puis dialysé sur hémodialyseur ou par ultrafiltration de manière à réduire la teneur en sels à une valeur équivalente à 0,15 M NaCl. L'éluat brut obtenu avant dialyse a ainsi une concentration protéique de 0,8 à 2,3 g/l et une teneur en facteur VII coagulant comprise entre 10 et 100 U/ml

A cette étape, on constate par un dosage approprié que la solution contient un mélange de FVII et FVII activé. Il est possible à ce stade d'activer davantage le FVII à l'aide de la célite ou de kaolin, connus pour activer le facteur XII en XIIa, qui, en retour, active le FVII. La concentration de célite utilisée est de 5 à 15 g/l de l'éluat dialysé précédemment obtenu. Cette nouvelle suspension est agitée à température ambiante puis centrifugée. Le surnageant de centrifugation est récupéré, le pH étant amené à 6 à 8.

La fraction ainsi obtenue peut contenir éventuellement des virus, en particulier des virus à membranes lipidiques : H.I.V., hépatites B et non A non B. Le procédé selon la présente invention comporte donc une étape d'élimination de ces virus par incubation du produit à inactiver avec un mélange contenant un solvant des membranes virales ainsi qu'un détergent non ionique. Le mélange inactivant utilisé de préférence est un mélange de Tween 80 à 1 % et de TnBP (n-tributyl phosphate) à 0,3 % ajouté à la solution protéique (poids/volume). La durée de l'inactivation est de 6 heures à la température de 24°C. après inactivation virale, le mélange doit être séparé des protéines par une méthode appropriée, en particulier par chromatographie sur gel de Q-Sepharose.

Il est possible d'inactiver les virus éventuellement contaminants soit au niveau de la matière première (prééluat du PPSB) soit au niveau du concentré de FVIIa final sans perte notable d'activité du produit.

La fraction ainsi obtenue présente une activité FVII sur la thromboplastine humaine (TpH) de l'ordre de 150 - 500 Unités/ml et une activité :

| | |
|---|---|
| FII | < 5 U/ml |
| FIX | < 10 U/ml |
| FX | < 10 U/ml |
| FVIII | < 1 U/ml |
| FV | < 1 U/ml |

pour un taux de protéine de l'ordre de 1 à 5 g/l et une activité spécifique FVIII tpH/mg.

Cette fraction est utilisable comme médicament, notamment dans le traitement des hémophiles A ayant un inhibiteur circulant, en particulier sous forme de composition pharmaceutique contenant, en outre, un agent stabilisant des protéines tel que l'albumine.

D'autres caractéristiques et avantages du procédé selon la présente invention apparaîtront à la lecture des exemples ci-après.

### Exemple 1

A partir de 700 l de surnageant cryo départ on récupère un volume de 140 l de prééluat de PPSB auquel on ajoute l'équivalent de 4 millions d'unités d'aprotinine. Le prééluat est filtré stérilement sur un filtre 0,2 » puis concentré par ultra-filtration sur cassettes 10 000 daltons jusqu'à un volume de 7 l (concentration environ 20 fois). Le concentrat présente une concentration protéique de 110 g/l et une osmolarité équivalente à 0,2 M NaCl et un pH de 7,5.

Le produit peut être congelé à cette étape en poches plasma de 2 l à -40°C pour traitement ultérieur.

Les poches sont décongelées à +37°C puis la solution est diluée avec 15 l de NaCl 0,18 M NaCl, on obtient ainsi un volume de 21 l de prééluat à une concentration protéique de 37 g/l. Le pH de la nouvelle solution est ajusté à 8 avec de la soude 1 N (soit 35 ml). On procède ensuite à l'adsorption sur phosphate tricalcique, ainsi 200 g de poudre sont ajoutés en pluie et on laisse en agitation pendant 40 minutes à température ambiante puis on centrifuge la suspension à un débit de 40 l/h. Le surnageant est éliminé et le culot est remis en suspension dans une solution de NaCl 0,15 M NaCl tamponnée à pH 8 avec du phosphate disodique.

Cette nouvelle suspension est de nouveau agitée pendant 15 minutes à température ambiante puis centrifugée à 40 l/h. Ce deuxième surnageant est éliminé et le culot est récupéré.

Le culot est ensuite remis en suspension dans 3 l de solution éluante de composition suivante :

| | | |
|---|---|---|
| . phosphate monosodique, | 0,25 M | |
| . phosphate disodique, | 0,25 M | |
| . NaCl, | 0,4 M, | pH 8 |

La suspension obtenue est agitée pendant 1 heure à température ambiante puis centrifugée, le surnageant (ou éluat brut) est récupéré et le culot écarté.

L'éluat brut est concentré et dialysé par ultrafiltration sur cassettes 10 K daltons jusqu'à un volume de 1 l et une osmolarité équivalente à 0,15 M NaCl ; le pH est ajusté 7 avec de l'acide acétique 0,1 N.

L'éluat ainsi concentré présente une activité en FVII de 245 U/ml pour un taux de protéines de 3 g/l ; le produit peut être congelé à -40°C en attente de l'étape d'inactivation virale.

### Exemple 2

A 1 l de concentré de facteur VII obtenu à l'exemple 1 est ajouté une solution de Twenn 1 % - TnBP 0,3 % à 24°C puis la solution est mise en agitation pendant 6 heures à la même température.

Le mélange inactivant est séparé des protéines par adsorption de celles-ci sur une résine échangeuse d'ions, comme par exemple le QAE-Sepharose qui a l'avantage de permettre une bonne rétention du facteur VII. Pour favoriser l'adsorption du FVII, il est possible de diluer la solution inactivée au demi avec de l'eau distillée de manière à abaisser la teneur en sels à une valeur équivalente à 0,075 M NaCl, le pH étant maintenu à 7.

Dans ces conditions, le mélange inactivant n'est pas retenu et les protéines sont éluées en augmentant la force ionique, le pH restant constant. Le gel prégonflé est équilibré avec un tampon phosphate mono-disodique 30 mM NaCl 0,075 M pH 7.

L'élution est réalisée avec le même tampon que celui de l'équilibrage mais contenant 0,5 M NaCl. L'éluat obtenu est dialysé et concentré jusqu'à isotonicité, le pH étant maintenu à 7.

Avant l'incorporation du concentré sur la colonne, il est possible d'ajouter à la solution 0,2 à 0,5 U/ml d'antithrombine III, ce qui permet d'inhiber la thrombine générée au cours de l'inactivation et la chromatographie sans que cela soit préjudiciable pour l'activité coagulante du facteur VII activé.

Ainsi, 2 l de solution inactivée sont adsorbés sur colonne de Q-Shepharose (1,5 l) à un débit de 100 cm/h. Le premier pic contient le mélange inactivant. Le deuxième pic ou pic d'élution permet de récupérer les proptéines adsorbées (soit un volume de 2 l environ). Le pic d'élution est concentré jusqu'à un volume de 1 l et dialysé jusqu'à isotonicité et pH 7.

A ce stade, il est possible de rajouter de 5 à 10 g/l d'albumine, ce qui permet la stabilisation des protéines.

On obtient ainsi un produit ayant une activité de 340 U FVII/ml et une activité spécifique de 115 U FVII/mg (avant addition d'albumine, qsp 10 mg/ml).

### Exemple 3

La présence de facteurs de coagulation a été recherchée dans le concentré, en particulier les facteurs II, IX, X, VII et VII activé. La méthode utilisée pour le dosage des facteurs est celle habituellement pratiquée, c'est-à-dire le raccourcissement du temps de coagulation d'un plasma déficient en présence d'un échantillon contenant le facteur manquant.

La présence de facteur VII activé dans la préparation est basée sur l'affinité différente du FVII et du FVII activé pour des thromboplastines d'origines diverses. Ainsi, le facteur VII activé a plus d'affinité pour la thromboplastine bovine (TpB) que pour la thromboplastine humaine (TpH).

Le rapport des activités VII(TpB)/VII(TpH) peut être une indication de l'activation du FVII. Un rapport supérieur à 1 révèle la présence de la forme activée de facteur VII. Les valeurs habituellement trouvées dans les préparations selon l'invention se situent entre 5 et 50, en général entre 5 et 15, ce qui révèle une grande teneur en facteur VII activé.

### Composition protéique moyenne du concentré obtenu à l'exemple 2

| Facteurs de coagulation | Unités/ml | Pourcentage |
|---|---|---|
| FVII(TpH) | 300 | 100 |
| FVII(TpB) | 2 500 | |
| FII | 2 | < 1 |
| FIX | 7 | < 2,5 |
| FX | 7 | < 2,5 |
| FVIII | 0,5 | < 0,2 |
| FV | 0,1 | < 0,04 |
| Thrombine | traces | - |

- Taux de protéines : 2 à 4 g/l
- Activité spécifique en unités FVII(TpH)/mg : 95 à 130

- Remarque :: Les pourcentages sont exprimés par rapport à l'activité FVII(TpH)

### Exemple 4 - Efficacité in vivo

Les essais ont été réalisés sur des chiens hémophiles A de race Beagle pesant 20 kg et présentant des taux plasmatiques de facteur VIII coagulant de 2 à 4 %.

Le temps de saignement est mesuré au niveau d'une plaie non délabrante de 2 mm de diamètre et de 1 mm de profondeur à l'aide d'un emporte-pièce dans la muqueuse de la paroi bucale interne.

Le produit est injecté par voie intra-veineuse (veine dorsale de l'avant-bras), les prélèvements sont effectués sur tubes plastiques citratés (10 % de solution à 3,1 g/l) ou sur EDTA via un cathéter veineux (veine dorsale de l'avant-bras ou jugulaire).

Le temps de saignement est mesuré amant injection sur une première plaie ; une seconde plaie est effectuée de la même manière 20 minutes après injection et le temps de saignement est mesuré.

### Raccourcissement du temps de saignement sur chiens hémophiles A après injection du concentré de FVIIa

| Dose injectée | | Temps de saignement | |
|---|---|---|---|
| U.FVII/ml plasma | U.FVII/kg | T 0 | T + 20' |
| 0,01 | 0,38 | 12' | 7' |
| 0,1 | 3,8 | 14' | 3-5' |
| 0,5 | 19 | 15' | 3-4' |
| 1,0 | 38 | 12' | 4' |

Le taux de facteur VII a été évalué en utilisant la thromboplastine humaine

Les résultats obtenus montrent que le concentré de FVIIa raccourcit le temps de saignement de façon significative à partir de 0,01 unité de FVII par ml de plasma, soit 0,38 unité par kg.

La dose efficace est de 4 unités de FVII par kg.

### REFERENCES

- Hedner U. and Kisiel W.; (1983), J. Clin. Invest., 71: 1836-1841. Use of Human Factor VIIa in the treatment of two Hemophilia A Patients with High-Titer Inhibitors.
- Seligsohn U., Kasper C.K., Osterud B. and Rapaport S.I.; (1979), Blood, 53: 828-837. Activated factor VII: presence in factor IX concentrates and persistence in the circulation after infusion.
- Soulier J.P., Blatrix C. and Steinbuch M.; (1964), Presse Med., 72: 1223. Fractions coagulantes contenant les facteurs de coagulation absorbables par le phosphate tricalcique.
- Van Deijk W.A., Van Dam-Mieras MCE., Muller A.D. and Hemker H.C.; (1983), Haemostasis, 13: 192-197.

## Revendications

1. Procédé de préparation d'une fraction riche en facteur VIIa, caractérisé en ce que :
- on concentre le prééluat du PPSB (ou complexe protrombique : prothrombine, proconvertine, facteur Stuart, facteur antihémophilique B) en présence d'un inhibiteur de la kallicréine,
- on fait adsorber le prééluat du PPSB par un absorbant ayant une affinité sélective pour les protéines se fixant au calcium choisi parmi les sels de métal divalent insoluble dans l'eau,
- on élue les protéines fixées par addition d'un tampon contenant des sels solubles capables de déplacer les protéines fixées,
- on récupère l'éluat concentré en facteur VIIa,
et en ce que les différentes étapes sont effectuées à une température comprise entre environ 15 et 30° C.

2. Procédé selon la revendication 1, caractérisé en ce que l'inhibiteur de la kallicréine est l'aprotinine.

3. Procédé selon l'une des revendications 1 et 2, caractérisé en ce que le sel de métal divalent est le phosphate tricalcique.

4. Procédé selon l'une des revendications 1 à 3, caractérisé en ce que les sels solubles sont choisis parmi :
- le mélange de phophate monosodique et disodique et NaCl,
- le citrate trisodique et NaCl.

5. Procédé selon l'une des revendications 1 à 4, caractérisé en ce que l'éluat est activé par un activateur du FVII.

6. Procédé selon la revendication 5, caractérisé en ce que l'activateur du FVII est choisi parmi la célite et le kaolin.

7. Procédé selon l'une des revendications 1 à 6, caractérisé en ce que l'éluat concentré en FVIIa ou le prééluat de PPSB est incubé avec un détergent non ionique et un solvant des membranes virales.

8. Procédé selon la revendication 7, caractérisé en ce que l'éluat concentré en FVIIa ou le prééluat de PPSB est incubé avec un mélange Tween et de n-tributyl phosphate.

9. Procédé selon l'une des revendications 7 et 8, caractérisé en ce que le détergent non ionique et le solvant des membranes virales sont éliminés par passage de la fraction incubée sur une colonne retenant sélectivement les protéines.

10. Procédé selon la revendication 9, caractérisé en ce que la colonne est une colonne QAE-Sepharose.

11. Procédé selon l'une des revendications 7 à 10, caractérisé en ce qu'après incubation la fraction obtenue est traitée par l'anti-thrombine III.

## Claims

1. Process for the preparation of a factor VIIa-rich fraction, characterized in that:
- the preeluate of PPSB (or prothrombin complex: prothrombin, proconvertin, Stuart factor, antihaemophilic factor B) is concentrated in the presence of a kallikrein inhibitor
- the preeluate of PPSB is caused to be adsorbed by an absorbent having a selective affinity for the proteins binding to calcium, which is chosen from the salts of a water-insoluble divalent metal,
- the bound proteins are eluted by adding a buffer containing soluble salts capable of displacing the bound proteins,
- the eluate concentrated with respect to factor VIIa is recovered,
and in that the various steps are carried out at a temperature of between about 15 and 30°C.

2. Process according to Claim 1, characterized in that the kallikrein inhibitor is aprotinin.

3. Process according to either of Claims 1 and 2, characterized in that the divalent metal salt is tricalcium phosphate.

4. Process according to one of Claims 1 to 3, characterized in that the soluble salts are chosen from:
- the mixture of monosodium and disodium phosphate and NaCl,
- trisodium citrate and NaCl.

5. Process according to one of Claims 1 to 4, characterized in that the eluate is activated by an activator of FVII.

6. Process according to Claim 5, characterized in that the activator of FVII is chosen from celite and kaolin.

7. Process according to one of Claims 1 to 6, characterized in that the eluate concentrated with respect to FVIIa or the preeluate of PPSB is incubated with a non-ionic detergent and a solvent for viral membranes.

8. Process according to Claim 7, characterized in that the eluate concentrated with respect to FVIIa or the preeluate of PPSB is incubated with a mixture of Tween and n-tributyl phosphate.

9. Process according to either of Claims 7 and 8, characterized in that the non-ionic detergent and the solvent for viral membranes are removed by passing the incubated fraction over a column selectively retaining proteins.

10. Process according to Claim 9, characterized in that the column is a QAE-sepharose column.

11. Process according to one of Claims 7 to 10, characterized in that after incubation, the fraction obtained is treated with anti-thrombin III.

## Patentansprüche

1. Verfahren zur Herstellung einer an Faktor VIIa reichen Fraktion, dadurch gekennzeichnet, daß man
- das Preeluat von PPSB (oder den Prothrombin-Komplex: Prothrombin, Proconvertin, Stuart-Faktor, Antihämophilie-B-Faktor) in Anwesenheit eines Inhibitors von Kallikrein konzentriert,
- das Preeluat von PPSB durch ein Absorbens mit einer selektiven Affinität für die Proteine, die sich an Calcium fixieren, adsorbiert, ausgewählt unter den Salzen zweiwertiger Metalle, die in Wasser unlöslich sind,
- die fixierten Proteine durch Zugabe eines Puffers eluiert, der lösliche Salze mit der Fähigkeit enthält, die fixierten Proteine zu verlagern,
- das an Faktor VIIa konzentrierte Eluat gewinnt,
und dadurch, daß die verschiedenen Stufen bei einer Temperatur zwischen etwa 15°C und 30°C durchgeführt werden.

2. Verfahren nach Anspruch 1, dadurch gekennzeichnet, daß der Inhibitor von Kallikrein Aprotinin ist.

3. Verfahren nach einem der Ansprüche 1 und 2, dadurch gekennzeichnet, daß das Salz des zweiwertigen Metalles Tricalciumphosphat ist.

4. Verfahren nach einem der Ansprüche 1 bis 3, dadurch gekennzeichnet, daß die löslichen Salze ausgewählt werden unter:
- der Mischung von Mononatriumphosphat und Dinatriumphosphat und NaCl,
- Trinatriumcitrat und NaCl.

5. Verfahren nach einem der Ansprüche 1 bis 4, dadurch gekennzeichnet, daß das Eluat durch einen Aktivator von FVII aktiviert wird.

6. Verfahren nach Anspruch 5, dadurch gekennzeichnet, daß der Aktivator von FVII unter Celit und Kaolin ausgewählt wird.

7. Verfahren nach einem der Ansprüche 1 bis 6, dadurch gekennzeichnet, daß das an Faktor VIIa konzentrierte Eluat oder das Preeluat von PPSB mit einem nicht-ionischen Detergens und einem Lösungsmittel für die viralen Membranen inkubiert wird

8. Verfahren nach Anspruch 7, dadurch gekennzeichnet, daß das an Faktor VIIa konzentrierte Eluat oder das Preeluat von PPSB mit einer Mischung von Tween und n-Tributyl-phosphat inkubiert wird.

9. Verfahren nach einem der Ansprüche 7 und 8, dadurch gekennzeichnet, daß das nicht-ionische Detergens und das Lösungsmittel für die viralen Membranen durch Passage der inkubierten Fraktion über eine Kolonne, die selektiv die Proteine zurückhält, entfernt werden.

10. Verfahren nach Anspruch 9, dadurch gekennzeichnet, daß die Kolonne eine QAE-Sepharose-Kolonne ist.

11. Verfahren nach einem der Ansprüche 7 bis 10, dadurch gekennzeichnet, daß nach der Inkubation die erhaltene Fraktion durch Anti-Thrombin III behandelt wird.
